# EUROPÄISCHE PATENTSCHRIFT

(11) **EP 3 167 088 B1**
(45) Veröffentlichungstag und Bekanntmachung des Hinweises auf die Patenterteilung: **29.01.2020**
(21) Anmeldenummer: 15748161.5
(22) Anmeldetag: 22.06.2015
(51) Int. Cl.: C22C 14/00

(54) **BIOKOMPATIBLES IMPLANTAT AUS EINER TITANLEGIERUNG**
BIOCOMPATIBLE IMPLANT MADE FROM A TITANIUM ALLOY
IMPLANT BIOCOMPATIBLE D'UN ALLIAGE DE TITANE

(30) Priorität: 08.07.2014 DE 102014010032
(43) Veröffentlichungstag der Anmeldung: 17.05.2017
(73) Patentinhaber: Wolter, Dietmar, 22955 Hoisdorf (DE)
(72) Erfinder: SIEMERS, Carsten, 38106 Braunschweig (DE); SIBUM, Heinz, 41515 Grevenbroich (DE)
(74) Vertreter: Gramm, Lins & Partner Patent- und Rechtsanwälte PartGmbB
(86) Internationale Anmeldenummer: PCT/DE2015/100252
(87) Internationale Veröffentlichungsnummer: WO 2016/004918

(56) Entgegenhaltungen:
- WO-A1-2014/143983
- JP-A- 2011 153 350
- US-A1- 2004 244 888
- Carlos Oldani ET AL: "Titanium as a Biomaterial for Implants" In: "Recent Advances in Arthroplasty, Edited by Dr. Samo Fokter", 27. Januar 2012 (2012-01-27), InTech, XP055215780, ISBN: 978-9-53-307990-5 DOI: 10.5772/27413,

## Beschreibung

Die Erfindung betrifft biokompatible Implantate aus einer Titanlegierung mit möglichst großer Festigkeit, die unter Ausschluss von Aluminium (Al), Vanadium (V), Kobalt (Co), Chrom (Cr), Nickel (Ni) und Zinn (Sn) hergestellt wird.

Auf Titanlegierungen wird in der Medizintechnik in der Regel dann zurückgegriffen, wenn aus ihr medizinische und/oder prothetische Implantate hergestellt werden sollen. Eine typischerweise verwendete Metalllegierung ist etwa Ti-Al6-V4, die eigentlich für die Luftfahrtindustrie entwickelt wurden. Von diesen Legierungen wurde später angenommen, dass sie zur Verwendung als Implantatmaterialien geeignet sind, da sie eine hinreichende mechanische Festigkeit besitzen und annehmbare Werte für die Biokompatibilität zu besitzen schienen.

Später wurde herausgefunden, dass viele der Materialien in Körperflüssigkeiten in einem gewissen Ausmaß korrodieren und dadurch Ionen freisetzen, die über einen längeren Zeitraum möglicherweise schädlich sein können. In der DE 690 08 507 T2 ist hierzu erläutert, dass angenommen wird, dass die korrodierenden Wirkungen der Körperflüssigkeiten sowohl auf chemische als auch auf elektrochemische Vorgänge zurückzuführen sind, wobei sich Korrosionsprodukte bilden, wenn bestimmte, gewöhnlich verwendete Metalllegierungen durch Korrosionsvorgänge im Körper Ionen bilden. Aluminiummetallionen sind zum Beispiel mit der Alzheimer-Krankheit in Verbindung gebracht worden und Vanadium, Kobalt, Nickel und Chrom stehen im Verdacht, toxisch oder karzinogen zu sein.

Im Allgemeinen ist es üblich, Implantat-Metalllegierungen zu passivieren. Die Passivierung erzeugt jedoch nur dünne, amorphe und schlecht haftende Oxidschutzfilme, die sich nicht als völlig wirksam beim Ausschalten der Bildung von Korrosionsprodukten im Körper erwiesen haben.

Die EP 1 211 993 B1 offenbart ein Fixationssystem für Knochen mit einem Kraftträger, in den ein durch Eindrehen einer Knochenschraube umformbares Element eingesetzt ist. Ein solches Fixationssystem dient dazu, Knochenbruchstücke miteinander zu verbinden. In der klinischen Anwendung hat sich eine Überlegenheit dieser Systeme gegenüber herkömmlichen Platten- und Nagelsystemen gezeigt. Auch für solche Fixationssysteme werden Titanwerkstoffe verwendet, die aufgrund ihrer, auf eine dünne Titanoxidschicht zurückzuführende Biokompatibilität eingesetzt werden. Im Vergleich zu Stählen oder anderen metallischen Werkstoffen haben Titanwerkstoffe hierfür auch ein geeignetes Eigenschaftsprofil, insbesondere hinsichtlich der Steifigkeit und Festigkeit, um im menschlichen Körper verwendet zu werden. Dazu werden in der Regel Titanwerkstoffe technischer Reinheit (CP-Titan) Grad 1S bis 4 oder die bereits genannten Legierungen Ti-Al6-V4 oder Ti-Al6-Nb7 eingesetzt. Weitere Titanwerkstoffe, hauptsächlich aus der Klasse der metastabilen β-Titanlegierungen (Ti-Mo15) und vereinzelt auch aus der Klasse der β-Titanlegierungen (TNZT) haben bereits eine medizintechnische Zulassung. Sie befinden sich jedoch noch in der Erprobung.

Allen Reintitansorten gemeinsam ist eine relativ geringe Festigkeit (Zugfestigkeit: Rₘ ≤ 600 MPa), was im Fall eines Einsatzes in der Osteosynthese zu relativ dicken Platten, Schrauben oder Nägeln führt. Für permanente Implantate, beispielsweise im Knie oder Hüftbereich, ist die Festigkeit von CP-Titan zu gering. In der Osteosynthese wird daher der Weg der Sandwichplatten gegangen, bei denen eine weiche Reintitansorte mit der Titanlegierung Ti-Al6-V4 formschlüssig verbunden wird, um so eine ausreichende Festigkeit zu garantieren. Zur Erhöhung der Winkelstabilität der Platten werden dabei häufig Schrauben verwendet, die ihr Gewinde beim Eindrehen in die Platten selbst schneiden, sodass eine bestehende Titanoxidschicht normalerweise beschädigt wird. Auch eine durch einen Anodisierungsprozess erzeugte dickere Titanoxidschicht schafft dabei keine Abhilfe.

Aus der Dissertation von Y. Müller (Diss. ETH Zürich Nr. 14542 (2002)) sind Untersuchungen bekannt geworden, die belegen, dass Metallionen bereits bei Raumtemperatur durch Risse in der Titanoxidschicht an die Oberfläche eines entsprechenden Bauteils gelangen können. Eine durch Einschrauben einer selbstschneidenden Gewindeschraube beschädigte Oxidschicht würde den Austritt der Metallionen noch erleichtern. Bezogen auf eine Verwendung der Legierung im menschlichen Körper würde dies bedeuten, dass entsprechend Aluminiumionen und/oder Vanadiumionen in den Blutkreislauf gelangen könnten. Im menschlichen Körper sind Bereiche mit einem sehr geringen Sauerstoffgehalt vorhanden. Wird dort Titan mit einer beschädigten Oxidschicht verwendet, kann sich dann keine neue Oxidschicht bilden, sodass eine erneute Passivierung der Legierung ausbleibt. Der Einsatz solcher Titanlegierungen ist daher insbesondere als Osteosynthesematerial nicht unbedingt optimal geeignet.

In der US 2004/0244888 A1 sind diverse Titanlegierungen offenbart, die Anteile von Sauerstoff und Eisen unter 1,0 Gew.% und einem Kohlenstoffgehalt von unter 0,08 Gew.% aufweisen. Diese Legierungen sind nicht dazu vorgesehen, auch für biokompatible Implantate verwendet zu werden, sodass der Ausschluss toxischer Elemente wie Aluminium, Vanadium, Kobalt, Chrom, Nickel und Zinn nicht grundsätzlich gewährleistet ist.

Aufgrund des relativ hohen Sauerstoffgehaltes wird dieser Werkstoff auch eine recht niedrige Ermüdungsbeständigkeit gewährleisten.

Die JP 2011-153350 A offenbart eine Titanlegierung mit hoher Festigkeit mit guten Walzeigenschaften, ohne dass hierzu die teuren Legierungselemente Vanadium, Molybdän oder Aluminium verwendet werden müssen. Diese Legierung kann für Automobilteile, für Golfschlägerköpfe oder schusssichere Platten verwendet werden. Eine Verwendung für biokompatible Implantate ist nicht vorgesehen.

In den Veröffentlichungen von Oldani und Dominguez, "Titanium as a Biomaterial for Implants" in: "Recent Advances in Arthroplasty, Edited by Dr. Samo Fokter" (ISBM: 978-9-53-307990-5). Wird die geschichtliche Entwicklung von Titanlegierungen für biokompatible Implantate beschrieben. Hiervon ausgehend soll eine Titanlegierung mit möglichst großer Festigkeit geschaffen werden, ohne dass dabei Legierungselemente verwendet werden, deren Toxizität bereits nachgewiesen ist oder die im Verdacht stehen, Krankheiten auszulösen.

Diese Aufgabe wird durch eine Titanlegierung gelöst, die unter Ausschluss von Aluminium (Al), Vanadium (V), Nickel (Ni), Chrom (Cr), Kobalt (Co) und Zinn (Sn) als Legierungselemente, neben unvermeidbaren Spurenmengen an Verunreinigungen, die in den Legierungsbestandteilen enthalten sind oder während der Herstellung aufgenommen wurden, deren Ausgangswerkstoff CP-Titan Grad 4 ist und die folgende Legierungsbestandteile in Gew.% aufweist:
a) 0,2 bis 1,5 % Sauerstoff (O),
b) 0,1 bis 1,5 % Eisen (Fe),
c) 0,01 bis 2 % Kohlenstoff (C),
d) weniger als 0,5 % Silizium (Si),
e) wahlweise zusätzlich weniger als 1 % Gold (Au) und/oder weniger als 1 % Niob (Nb) und/oder weniger als 1 % Molybdän (Mo) und/oder weniger als 1 % Zirconium (Zr) und/oder weniger als 1,5 % Stickstoff (N) und/oder weniger als 0,2 % Wasserstoff (H),
f) Rest Titan (Ti).

Auch wenn Spurenmengen an Verunreinigungen niemals vermeidbar sind, wird die Festigkeitssteigerung bei der Reintitansorte dadurch erreicht, dass nur bereits im menschlichen Körper enthaltene Legierungsbestandteile verwendet werden.

Vorzugsweise werden nur diese Bestandteile verwendet und dann insbesondere bevorzugt 0,4 Gew.% Sauerstoff (O) und/oder 0,5 Gew.% Eisen (Fe) und/oder 0,08 Gew.% Kohlenstoff (C) verwendet.

Zusätzlich können in der Titanlegierung zur Festigkeitssteigerung aber auch noch solche Legierungsbestandteile verwendet werden, die keine bekannten negativen Auswirkungen auf den Körper haben, wie beispielsweise Gold, Molybdän, Niob, Silizium und Zirconium. Der Anteil an Gold (Au) ist vorzugsweise kleiner als 1 Gew.% und beträgt insbesondere vorzugsweise 0,1 Gew.%.

Niob (Nb) wird vorzugsweise zusätzlich mit einem Anteil von weniger als 1 Gew.%, und insbesondere vorzugsweise mit einem Anteil von 0,1 Gew.% verwendet.

Molybdän (Mo) wird vorzugsweise zusätzlich mit einem Anteil von weniger als 1 Gew.% und insbesondere mit einem Anteil von 0,1 Gew.% verwendet.

Zirconium (Zr) wird vorzugsweise mit einem Anteil von weniger als 1 Gew.% und insbesondere mit einem Anteil von 0,1 Gew.% verwendet. Stickstoff (N) kann zusätzlich mit einem Anteil von weniger als 1,5 Gew.% und insbesondere mit einem Anteil von weniger als 0,4 Gew.% und dabei insbesondere mit einem Anteil von 0,2 Gew.% verwendet werden. Silizium (Si) wird vorzugsweise mit einem Anteil von 0,05 Gew.% verwendet.

Auch Wasserstoff (H) kann mit einem Anteil von vorzugsweise weniger als 0,2 Gew.% in der erfindungsgemäßen Legierung verwendet werden.

Die Elemente können kumulativ in der Legierung vorhanden sein. Einzelne Elemente können aber auch vollständig fehlen, je nachdem, welche Festigkeit im speziellen Anwendungsfall benötigt wird. Grundsätzlich ausgeschlossen muss aber die Verwendung der Elemente Aluminium (Al), Vanadium (V) und Zinn (Sn) sein, wobei auch hier natürlich nicht ausgeschlossen werden kann, dass diese als Verunreinigungen in anderen Legierungsbestandteilen unvermeidbar sind und deshalb als Spurenelement toleriert werden müssen.

Die Legierung kann bevorzugt für ein "intelligentes Implantat mit Verblockungstechnik", wie es beispielsweise in der EP 1 211 993 B1 oder der EP 1 143 867 B1 beschrieben ist, verwendet werden. Auch kann die Legierung zur Herstellung von Knochenschrauben- und Knochennagelmaterial eingesetzt werden.

Der Ausgangswerkstoff ist CP-Titan Grad 4, dessen zugelassene Maximalgehalte an Begleitelementen in der für die Medizintechnik gültigen Spezifikation ASTM F-67 festgelegt sind. Die nachfolgende Tabelle gibt eine mögliche Zusammensetzung der erfindungsgemäßen Titanlegierung an, wobei die Angaben in Gew.% erfolgen:

| **Werkstoff** | **Ti** | **O** | **Fe** | **C** | **Au** | **Nb** | **Mo** | **Zr** | **N** | **Si** |
|---|---|---|---|---|---|---|---|---|---|---|
| CP Ti Grad 4⁺ | Rest | 0,4 | 0,5 | 0,08 | | | | | | |
| Gold-Titan Grad 4⁺ | Rest | 0,4 | 0,5 | 0,08 | 0,1 | | | | | |
| Gold-Titan Grad 4⁺Nb | Rest | 0,4 | 0,5 | 0,08 | 0,1 | 0,1 | | | | |
| Gold-Titan Variante 1 | Rest | 0,4 | 0,5 | 0,08 | 0,1 | 0,1 | 0,1 | | | |
| Gold-Titan Variante 2 | Rest | 0,4 | 0,5 | 0,08 | 0,1 | 0,1 | 0,1 | 0,1 | | |
| Gold-Titan Variante 3 | Rest | 0,4 | 0,5 | 0,08 | 0,1 | 0,1 | 0,1 | 0,1 | 0,2 | |
| Gold-Titan Variante 4 | Rest | 0,4 | 0,5 | 0,08 | 0,1 | 0,1 | 0,1 | 0,1 | 0,2 | 0,05 |

Um abzuschätzen, welche Gehalte an Legierungselementen sich für den geplanten Einsatzfall eignen, wurden zunächst verschiedene binäre Legierungen untersucht. Neben dem Gefüge und der Härte wurde insbesondere die Schlagzähigkeit bei Raumtemperatur von Ti-O (0,2 bis 1,5), Ti-Fe (0,2 bis 1,5) und Ti-Nb (0,1 bis 2) untersucht (Angaben in Gew.%).

Es hat sich dann herausgestellt, dass durch die Zugabe von Gold einerseits eine weitere Mischkristallverfestigung des Werkstoffs erreicht wird und andererseits eine überraschenderweise kornfeinende Wirkung durch Ausscheiden von zusätzlichen Teilchen im Mikrometerbereich, vorwiegend auf den Korngrenzen, erfolgte. Dies ist deshalb überraschend, weil das binäre Phasendiagramm Ti-Au etwas anderes vorhersagt. Diese Wirkung wird vermutlich aufgrund der geringen Löslichkeit von Gold in Titan erreicht.

Niob sorgt ebenfalls für eine geringe zusätzliche Mischkristallverfestigung.

Aufgrund möglicher negativer Auswirkungen auf den menschlichen Körper sind die in der vorstehenden Tabelle enthaltenen ersten drei Legierungen zu bevorzugen, auch wenn die Festigkeit etwas geringer ist als die der Gold-Titanvarianten 1 bis 4.

Erfindungsgemäß wird auf Legierungselemente zurückgegriffen, die für Titanlegierungen bisher selten oder gar nicht eingesetzt wurden. Der eingetretene Erfolg war damit nicht vorhersehbar. Vielmehr müssen alle Mechanismen eingesetzt werden, die zu einer Verfestigung führen können, wie beispielsweise die Mischkristallhärtung, die Feinkornhärtung oder die Verformungsverfestigung.

Die Legierungsherstellung im Labormaßstab erfolgte in einem Plasma-Lichtbogenofen, das Erschmelzen und Vergießen war problemlos möglich. Anschließend erfolgten eine Lösungsglühung unter Schutzgas (Ar 99,998), eine Gefügeanalyse und eine Härteprüfung zur Abschätzung der mechanischen Eigenschaften. Für die Legierung CP-Ti Grad 4⁺ wurden Umformversuche (statisch: Umformgrad = 0,9; dynamisch: Umformgrad = 0,3) durchgeführt, mit denen bewiesen werden konnte, dass sich der erfindungsgemäße Titanwerkstoff warm umformen lässt, was eine Voraussetzung für den technischen Einsatz ist. Aufgrund des gerätebedingten Umformgrads von etwa 0,3 im dynamischen Umformversuch konnte durch eine Rekristallisationsglühung kein Feinkorn erzielen lassen. Aufgrund zusätzlicher Mischkristallverfestigung und gegebenenfalls durch das Entstehen einer zweiphasigen Titanlegierung sollte die Festigkeit der weiteren vorstehend beschriebenen Lösungsvarianten aber in jedem Fall oberhalb der Festigkeit von CP-Ti Grad 4⁺ liegen.

Ein Beispiel für die Legierungsherstellung zeigt folgende Tabelle:

| **Element** | **wt.-% [soll]** | **300g [soll]** | **Vorlegierungen** | **300g [ist]** | **wt.-% [ist]** |
|---|---|---|---|---|---|
| Titan | 99,02 (-0,48) | 300,000 | CP-Titan Grad 4 | 300,003 | 99,02 |
| Sauerstoff | 0,40 (+0,10) | 1,206 (+0,301) | TiO₂ 99,98% | 0,301 | 0,40 |
| Eisen | 0,50 (+0,31) | 1,507 (+0,935) | 99,98% | 0,936 | 0,50 |
| Kohlenstoff | 0,08 (+0,07) | 0,241 (+0,211) | 99,995% | 0,211 | 0,08 |
| | | | | | |
| Summe | 100,00 | 301,447 | | 301,451 | 100,00 |

Als Vorlegierung wurde CP-Ti Grad 4 der Firma Daido Steel (FJ2-FJ3, Heat No. TN831G) als Stangenmaterial in einem Durchmesser von 8 mm verwendet. Die chemische Zusammensetzung wurde dem entsprechenden Analysezertifikat entnommen. Für die Erhöhung des Sauerstoff- und Kohlenstoffgehalts wurden entsprechende Pulver (TiO₂ und Graphit) abgewogen und zur Vermeidung von Abblasverlusten in eine Titanfolie verpackt, die zwischen die Titanstangen platziert wurde. Der Titangehalt der Titanfolie betrug 99,6 % und lag damit etwas oberhalb der eingesetzten Vorlegierung. Die daraus resultierenden geringfügigen Abweichungen in der chemischen Zusammensetzung wurden vernachlässigt. Weil das Gewicht der Titanfolie lediglich 2,22 g bei einem Gesamtgewicht von 301,45 g betrug und die chemische Zusammensetzung der Folie annähernd derjenigen des eingesetzten CP-Ti Grad 4 entsprach, erscheint die Vernachlässigung vertretbar. Eisen wurde in Granulatform hinzugefügt.

Die nachfolgende Tabelle zeigt die gemessenen Härten (Verfahren: Vickers HV10/15) und die daraus abgeschätzten Zugfestigkeiten. Zum Vergleich sind die Legierungen Ti-Al6-V4, Ti-Al6-V4 ELI sowie die metastabile β-Titanlegierung Ti-Mo15 im Lösungsgeglühten und abgeschreckten Zustand (LG) sowie im ausscheidungsgehärteten Zustand (AG) dargestellt.

| **Werkstoff** | **HV10/15** | **Rₘ /MPa** |
|---|---|---|
| CP-Ti-Grad 4 | 221 | 570 |
| CP-Ti-Grad 4⁺ | 274 | 760 |
| Gold-Titan-Grad 4⁺ | 295 | 840 |
| Gold-Titan-Grad 4⁺ Nb | 300 | 860 |
| Ti-Al6-V4 | 290 - 340*) | 820 - 1000*) |
| Ti-Al6-V4 ELI | 285 - 330*) | 800 - 960*) |
| Ti-Mo15 LG | 215 | 550 |
| Ti-Mo15 AG | 429 | 1320 |

| | | |
|---|---|---|
| *) je nach Gefügezustand | | |

Die Härte der erfindungsgemäßen Reintitanvarianten CP-Ti Grad 4+ und Gold-Titan Grad 4+ liegt um etwa 20 % höher als die Härte der härtesten Reintitansorte CP-Ti Grad 4 und nur um etwa 10 % unterhalb oder an der Untergrenze der Härte der bisher hauptsächlich eingesetzten Titanlegierungen Ti-Al6-V4 und Ti-Al6-V4 ELI.

Die nachfolgende Tabelle zeigt den Einfluss der Umformtemperatur (Umformverfahren: Rundkneten, Umformgrad 0,3) und einer anschließenden Rekristallisationsglühung auf die Härte des Werkstoffs CP-Ti Grad 4⁺. Dabei wurden fünf Eindrücke gesetzt (Pos. 1 bis 5).

| **Werkstoff** | **Pos. 1** | **Pos. 2** | **Pos. 3** | **Pos. 4** | **Pos. 5** | **HV10/15** |
|---|---|---|---|---|---|---|
| CP-Ti-Grad 4⁺ 600°C | 271 | 276 | 282 | 279 | 275 | 276 ± 4 |
| CP-Ti-Grad 4⁺ 600°C RK | 262 | 265 | 258 | 262 | 260 | 261 ± 3 |
| CP-Ti-Grad 4⁺ 800°C | 265 | 267 | 263 | 263 | 263 | 264 ± 2 |
| CP-Ti-Grad 4⁺ 800°C RK | 248 | 257 | 258 | 263 | 262 | 258 ± 6 |
| CP-Ti-Grad 4⁺ 900°C | 272 | 270 | 270 | 276 | 279 | 273 ± 4 |
| CP-Ti-Grad 4⁺ 900°C RK | 262 | 262 | 265 | 254 | 281 | 265 ± 9 |

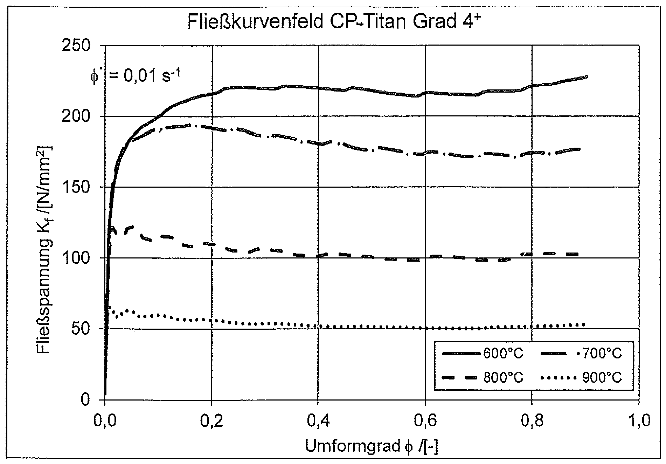

Die einzige Figur zeigt ein Fließkurvenfeld. Aufgetragen sind die quasi statischen Fließkurven der Legierung CP-Ti Grad 4⁺ in Abhängigkeit der Temperatur. Wenn ein Umformgrad von 0,9 erreicht war, wurde der Versuch abgebrochen. Zu einem Bruch der Proben ist es nicht gekommen. Das Fließkurvenfeld lässt deutlich erkennen, dass die untersuchte Legierung CP-Ti Grad 4⁺ schmiedbar ist.

## Patentansprüche

1. Biokompatibles Implantat aus einer Titanlegierung, die unter Ausschluss von Aluminium (Al), Vanadium (V), Kobalt (Co), Chrom (Cr), Nickel (Ni) und Zinn (Sn) als Legierungselemente, neben unvermeidbaren Spurenmengen an Verunreinigungen, die in den Legierungsbestandteilen enthalten sind oder während der Herstellung aufgenommen wurden, die folgende Legierungsbestandteile in Gew.% aufweist:
a) 0,2 bis 1,5 % Sauerstoff (O),
b) 0,1 bis 1,5 % Eisen (Fe),
c) 0,01 bis 2 % Kohlenstoff (C),
d) weniger als 0,5 % Silizium (Si),
e) wahlweise zusätzlich weniger als 1 % Gold (Au) und/oder weniger als 1 % Niob (Nb) und/oder weniger als 1 % Molybdän (Mo) und/oder weniger als 1 % Zirconium (Zr) und/oder weniger als 1,5 % Stickstoff (N) und/oder weniger als 0,2 % Wasserstoff (H),
f) Rest Titan (Ti).

2. Biokompatibles Implantat nach Anspruch 1, **gekennzeichnet durch** 0,4 % Sauerstoff (O) in der Titanlegierung.

3. Biokompatibles Implantat nach Anspruch 1 oder 2, **gekennzeichnet durch** 0,5 % Eisen (Fe) in der Titanlegierung.

4. Biokompatibles Implantat nach einem der Ansprüche 1 bis 3, **gekennzeichnet durch** 0,08 % Kohlenstoff (C) in der Titanlegierung.

5. Biokompatibles Implantat nach Anspruch 1, **gekennzeichnet durch** 0,1 % Gold (Au) in der Titanlegierung.

6. Biokompatibles Implantat nach Anspruch 1, **gekennzeichnet durch** 0,1 % Niob (Nb) in der Titanlegierung.

7. Biokompatibles Implantat nach Anspruch 1, **gekennzeichnet durch** 0,1 % Molybdän (Mo) in der Titanlegierung.

8. Biokompatibles Implantat nach Anspruch 1, **gekennzeichnet durch** 0,1 % Zirconium (Zr) in der Titanlegierung.

9. Biokompatibles Implantat nach Anspruch 1, **gekennzeichnet durch** weniger als 0,4 % Stickstoff (N) in der Titanlegierung.

10. Biokompatibles Implantat nach Anspruch 1, **gekennzeichnet durch** 0,2 % Stickstoff (N) in der Titanlegierung.

11. Biokompatibles Implantat nach Anspruch 1, **gekennzeichnet durch** 0,05 % Silizium (Si) in der Titanlegierung.

12. Biokompatibles Implantat nach einem der vorherigen Ansprüche 1 bis 11, das ein prothetisches Implantat ist.

## Claims

1. Biocompatible implant made from a titanium alloy which, with exclusion of aluminium (Al), vanadium (V), cobalt (Co), chromium (Cr), nickel (Ni) and tin (Zn) as alloying elements, comprises the following alloying constituents in % by weight besides unavoidable trace quantities of impurities which are present in the alloying constituents or were incorporated during production:
a) 0.2% to 1.5% of oxygen (0),
b) 0.1% to 1.5% of iron (Fe),
c) 0.01% to 2% of carbon (C),
d) less than 0.5% of silicon (Si),
e) optionally additionally less than 1% of gold (Au) and/or less than 1% of niobium (Nb) and/or less than 1% of molybdenum (Mo) and/or less than 1% of zirconium (Zr) and/or less than 1.5% of nitrogen (N) and/or less than 0.2% of hydrogen (H),
f) the remainder being titanium (Ti).

2. Biocompatible implant according to Claim 1, **characterized by** 0.4% of oxygen (0) in the titanium alloy.

3. Biocompatible implant according to Claim 1 or 2, **characterized by** 0.5% of iron (Fe) in the titanium alloy.

4. Biocompatible implant according to any of Claims 1 to 3, **characterized by** 0.08% of carbon (C) in the titanium alloy.

5. Biocompatible implant according to Claim 1, **characterized by** 0.1% of gold (Au) in the titanium alloy.

6. Biocompatible implant according to Claim 1, **characterized by** 0.1% of niobium (Nb) in the titanium alloy.

7. Biocompatible implant according to Claim 1, **characterized by** 0.1% of molybdenum (Mo) in the titanium alloy.

8. Biocompatible implant according to Claim 1, **characterized by** 0.1% of zirconium (Zr) in the titanium alloy.

9. Biocompatible implant according to Claim 1, **characterized by** less than 0.4% of nitrogen (N) in the titanium alloy.

10. Biocompatible implant according to Claim 1, **characterized by** 0.2% of nitrogen (N) in the titanium alloy.

11. Biocompatible implant according to Claim 1, **characterized by** 0.05% of silicon (Si) in the titanium alloy.

12. Biocompatible implant according to any of the preceding Claims 1 to 11, which is a prosthetic implant.

## Revendications

1. Implant biocompatible en un alliage de titane contenant, à l'exclusion de l'aluminium (Al), du vanadium (V), du cobalt (Co), du chrome (Cr), du nickel (Ni) et de l'étain (Sn) en tant qu'éléments d'alliage, outre des traces inévitables d'impuretés contenues dans les constituants d'alliage ou absorbées lors de la fabrication, les constituants d'alliage suivants en % en poids :
a) 0,2 à 1,5 % d'oxygène (O),
b) 0,1 à 1,5 % de fer (Fe),
c) 0,01 à 2 % de carbone (C),
d) moins de 0,5 % de silicium (Si),
e) au choix en supplément moins de 1 % d'or (Au) et/ou moins de 1 % de niobium (Nb) et/ou moins de 1 % de molybdène (Mo) et/ou moins de 1 % de zirconium (Zr) et/ou moins de 1,5 % d'azote (N) et/ou moins de 0,2 % d'hydrogène (H),
f) le reste étant du titane (Ti).

2. Implant biocompatible selon la revendication 1, **caractérisé par** 0,4 % d'oxygène (O) dans l'alliage de titane.

3. Implant biocompatible selon la revendication 1 ou 2, **caractérisé par** 0,5 % de fer (Fe) dans l'alliage de titane.

4. Implant biocompatible selon l'une des revendications 1 à 3, **caractérisé par** 0,08 % de carbone (C) dans l'alliage de titane.

5. Implant biocompatible selon la revendication 1, **caractérisé par** 0,1 % d'or (Au) dans l'alliage de titane.

6. Implant biocompatible selon la revendication 1, **caractérisé par** 0,1 % de niobium (Nb) dans l'alliage de titane.

7. Implant biocompatible selon la revendication 1, **caractérisé par** 0,1 % de molybdène (Mo) dans l'alliage de titane.

8. Implant biocompatible selon la revendication 1, **caractérisé par** 0,1 % de zirconium (Zr) dans l'alliage de titane.

9. Implant biocompatible selon la revendication 1, **caractérisé par** moins de 0,4 % d'azote (N) dans l'alliage de titane.

10. Implant biocompatible selon la revendication 1, **caractérisé par** 0,2 % d'azote (N) dans l'alliage de titane.

11. Implant biocompatible selon la revendication 1, **caractérisé par** 0,05 % de silicium (Si) dans l'alliage de titane.

12. Implant biocompatible selon l'une des revendications précédentes 1 à 11, qui est un implant prothétique.
